# EUROPEAN PATENT SPECIFICATION

(11) **EP 2 231 327 B1**
(45) Date of publication and mention of the grant of the patent: **16.10.2013**
(21) Application number: 08866807.4
(22) Date of filing: 24.12.2008
(51) Int. Cl.: B01J 21/06, C07C 67/08, B01J 27/053

(54) **CATALYST COMPOSITION INCLUDING ZIRCONIUM COMPOUNDS FOR ESTERFICATION REACTION AND METHOD FOR PREPARING ESTER COMPOUNDS**
KATALYSATORZUSAMMENSETZUNG MIT ZIRKONIUMVERBINDUNGEN ZUR VERESTERUNGSREAKTION UND VERFAHREN ZUR HERSTELLUNG VON ESTERVERBINDUNGEN
COMPOSITION CATALYSANTE COMPRENANT DES COMPOSÉS DE ZIRCONIUM DESTINÉS À CRÉER UNE RÉACTION D'ESTÉRIFICATION, ET MÉTHODE DE PRÉPARATION DE COMPOSÉS D'ESTER

(30) Priority: 27.12.2007 KR 20070139034
(43) Date of publication of application: 29.09.2010
(73) Proprietor: LG Chem, Ltd., Seoul 150-721 (KR)
(72) Inventor: CHOI, Dai-Seung, Daejeon Metropolitan City 305-759 (KR); KANG, Yu-Chan, Seoul 150-030 (KR); CHUN, Sung-Ho, Daejeon Metropolitan City 302-772 (KR); KIM, Heon, Daejeon Metropolitan City 305-770 (KR); YOO, Dong-Woo, Daejeon Metropolitan City 305-729 (KR)
(74) Representative: Goddar, Heinz J.
(86) International application number: PCT/KR2008/007669
(87) International publication number: WO 2009/084862

(56) References cited:
- WO-A1-2004/099115
- JP-A- 6 321 878
- US-A- 3 510 254
- US-A- 3 551 095
- US-A- 4 042 672
- US-A- 5 191 139
- US-A- 5 614 650
- US-A- 5 866 710
- US-A1- 2007 004 936
- US-B1- 6 372 929
- US-B1- 6 448 198
- YADAV G D ET AL: "Heterogeneous catalysis in esterification reactions: preparation of phenethyl acetate and cyclohexyl acetate by using a variety of solid acidic catalysts", INDUSTRIAL & ENGINEERING CHEMISTRY RESEARCH, AMERICAN CHEMICAL SOCIETY, US, vol. 33, no. 9, 1 January 1994 (1994-01-01), pages 2198-2208, XP009124575, ISSN: 0888-5885, DOI: 10.1021/IE00033A025

## Description

### [Technical Field]

The present invention relates to an esterification catalyst composition that includes a zirconium compound and a method for producing an ester compound by using the same.

### [Background Art]

An esterification reaction that is one of representative reactions of organic synthesis is an important reaction that has high utility value in views of environmentally friendly chemical processes, and many studies thereof have been reported.

For example, in order to synthesize biodiesel having high quality from vegetable-oil including an oleic acid and a stearic acid, an esterification reaction is applied using a catalyst, an importance of which has been embossed (Nature 438, 178, 2005).

In general, the esterification reaction frequently uses a coupling agent and an auxiliary activator in an amount of 1 equivalent or more on the basis of reactants, and after the reaction, since a great amount of side products are generated, an additional purification process such as distillation or recrystallization is required.

In addition, in a real reaction, if any one of a carboxylic acid and alcohol is not used in an excessive amount, there is a problem in that it is impossible to obtain efficiently ester (for example, see Synthesis, 1978 p. 929, Chem. Lett, 1977 p.55, Chem. Lett. 1981 p.663, Tetrahedron. Lett. 28, 1987 p.3713, J. Org. Chem. 56, 1991 p.5307). However, as described above, it is ideal to directly perform esterification from the carboxylic acid and alcohol in the same or similar mole number while the reactant is used in an excessive amount.

Accordingly, currently, by using the carboxylic acid and alcohol in almost the same mole number, a catalyst for synthesizing ester has been developed.

For example, Korean Patent Application Laid-Open No. 2003-0042011 relates to a method for producing an ester condensate, and a synthesis reaction of a monomer ester or thioester, or polyester or polythioester by using a tetravalent hafnium compound represented by hafnium chloride (IV), particularly, hafnium chloride (IV) (THF)₂ or hafnium (IV)t-butoxide as a (poly)condensation catalyst.

In addition, there is an example of esterification reaction by using a catalyst that includes zirconium(IV) compound and/or hafnium(IV) compound and iron compound and/or gallium compound. At this time, as the zirconium(IV) compound, a compound that is represented by Zr(OH)ₐ(OR1)_{b} (R1 is an acyl group or an alkyl group, a and b are each an integer in the range of 0 or 1∼4, and a + b = 4), or zirconium(IV) halogenate is used.

However, even though the hafnium compound, the zirconium halogenate, and the zirconium compound have excellent performance, there is a disadvantage in that it is not well dissolved in a nonpolar solvent such as heptane, octane, and toluene at normal temperature because of a characteristic of inorganic salt compound. In addition, after the reaction is finished, while the post-treatment is performed, catalyst residuals remain on a wall of a reactor. Thus, there is a problem in that it is difficult to remove the residuals.

In addition, Korean Patent Application Laid-Open No. 2005-0050549 discloses an example of production of carboxylic acid ester by reacting a carboxylic acid and a , monohydroxy compound in the presence of a catalyst after the zirconium catalyst is produced by reacting the monohydroxy compound and a Zr(OR)₄ type of (R is an alkyl group or an aryl group) zirconium compound with each other. At this time, a ligand that is bonded to a zirconium element is a form where an oxygen atom is included in one single molecule (monodentate).

US 3,551,095A discloses a process for producing carbonate hydrous zirconia substantially free from iron which comprises reacting barium zirconate containing iron as an impurity with an aqueous solution of an alkali bicarbonate to form a soluble zirconium complex, CO₂ being introduced into the reactants, filtering of insolubles containing iron, hydrolyzing the soluble zirconium complex and filtering off and washing the carbonated hydrous zirconia thus produced.

US 3,510,254A discloses a process for making a carbonated hydrate of zirconium in readily filterable form which comprises dissolving the crystalline oxychloride of said metal in a solvent comprising water, reacting said oxychloride with an effective amount of a sulfate donor to convert said oxychloride to the corresponding sulfate, suspending said sulfate particles in water and reacting same with the carbonate to convert the sulfate to the corresponding carbonated hydrate.

US 4,042,672A discloses a process for preparing carbonated zirconium oxide hydrate which comprises adding zirconium oxychloride . 8 H₂O to a saturated aqueous ammonium chloride and/or alkali chloride solution which contains at least equimolecular amounts of ammonium carbonate and/or alkali carbonate, separating the resulting precipitated reaction product and washing said product chloride-free with water.

### [Disclosure]

### [Technical Problem]

The present invention has been made keeping in mind the problems of low solubility to a nonpolar solvent of the known esterification catalyst and a difficulty in application to a mass synthesis process because the esterification is performed under an inert gas atmosphere, and an object of the present invention is to provide an esterification catalyst composition including a zirconium compound, and a method for producing an ester compound using the same.

### [Technical Solution]

Hereinafter, the present inveniton will be described in detail.

An esterification catalyst composition according to the present invention is provided in claim 1, a method in claim 3 and 8. Preferred embodiments are disclosed in the subclaims.

### [Advantageous Effects]

An inventive esterification catalyst composition has high solubility to a nonpolar solvent and is capable of reducing degradation in catalyst by moisture adsorption, thus an esterification reaction may be desirably performed under a dry inert gas atmosphere such as nitrogen gas and in a state exposed to an atmosphere. Accordingly, the composition may be applied to a mass production process.

### [Best Mode]

In addition, the composition comprises a zirconium carbonate basic hydrate ([3ZrO₂ · CO₂ · (H₂O)ₓ]). At this time, X of the zirconium compounds is a variable integer in the range of 1 to 12 according to a reaction condition and an atmosphere around a zirconium atom unit.

The esterification catalyst composition according to the present invention may be used while being carried in a solid phase or a particulate support phase. At this time, it is preferable that the particulate support is one or more selected from the group consisting of silica, titania, silica/cromia, silica/cromia/titania, silica/alumina, aluminium phosphate gel, silanated silica, silica hydro gel, montmorilonite clay and zeolite.

According to a method for producing an ester compound according to the present invention, by esterifying alcohol and carboxylic acid compounds by using the esterification catalyst composition, the ester compound may be produced.

Alcohol that is used in the production method of the ester compound according to the present invention includes compounds that are represented by the following Formulas 2 to 7.

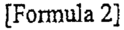

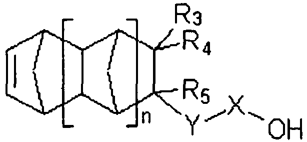

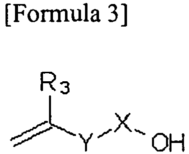

In Formulas 2 and 3,
n is an integer in the range of 0 to 4,
Y is a direct bond, or is selected from the group consisting of -C(O)-O-, -O-C(O)-, -C(O)N(R₃)-, -OC(O)N(R₃)-, - N(R₃)-, -C(O)-, -SO₂-, -SO₃- and -OSO₂-,
X is selected from the group consisting of a direct bond; -(OCH₂)ₘ- (m is an integer in the range of 0 to 10); substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkynylene having 2 to 20 carbon atoms;
R₃, R₄ and R₅ are each independently selected from the group consisting of hydrogen; halogen; straight- or branched-chained alkyl having 1 to 20 carbon atoms; straight- or branched-chained alkenyl or vinyl having 2 to 20 carbon atoms; alkynyl having 2 to 20 carbon atoms; cycloalkyl that is unsubstituted or substituted with hydrocarbons and has 3 to 12 carbon atoms; aryl that is unsubstituted or substituted with hydrocarbons and has 6 to 40 carbon atoms; and aralkyl that is unsubstituted or substituted with hydrocarbons and has 7 to 15 carbon atoms,
R₃, R₄ and R₅ are each independently selected from the group consisting of hydrogen; halogen; straight- or branched-chained alkyl having 1 to 20 carbon atoms; straight- or branched-chained alkenyl or vinyl having 2 to 20 carbon atoms; alkynyl having 2 to 20 carbon atoms; cycloalkyl that is unsubstituted or substituted with hydrocarbons and has 5 to 12 carbon atoms; aryl that is unsubstituted or substituted with hydrocarbons and has 6 to 40 carbon atoms; and aralkyl that is unsubstituted or substituted with hydrocarbons and has 7 to 15 carbon atoms,

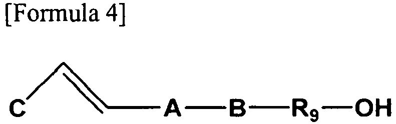

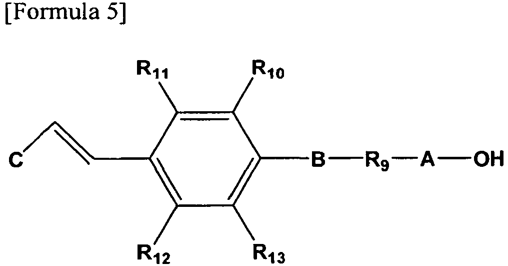

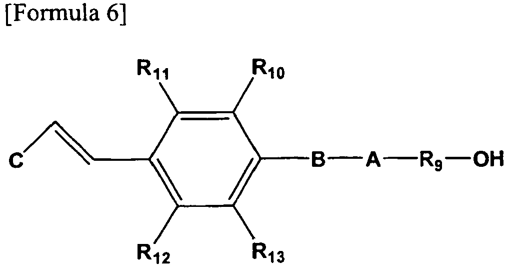

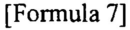

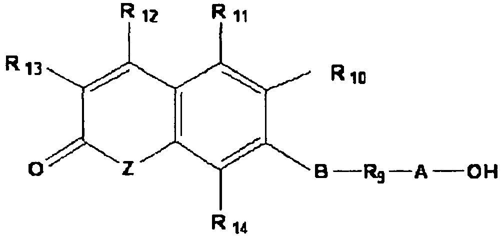

In the compounds that are represented by Formulas 4 to 7,
A is a direct bond; or any one selected from the group consisting of substituted or unsubstituted alkylene having 1 to 20 carbon atoms; carbonyl [-C(O)-]; carboxy [-C(O)O- or -OC(O)-]; and substituted or unsubstituted arylene having 6 to 40 carbon atoms,
B is a direct bond; oxygen, sulfur or -NH-,
Z is oxygen or sulfur,
R₉ is a direct bond; or any one selected from the group consisting of substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkynylene having 2 to 20 carbon atoms,
R₁₀, R₁₁, R₂₁ R₁₃ and R₁₄ are each independently selected from the group consisting of hydrogen; substituted or unsubstituted alkyl having 1 to 20 carbon atoms; substituted or unsubstituted alkoxy having 1 to 20 carbon atoms; substituted or unsubstituted aryloxy having 6 to 30 carbon atoms; and substituted or unsubstituted aryl having 6 to 40 carbon atoms, and
C is a compound that includes hetero aryl including Groups 14, 15, or 16 heteroatoms, for example, heteroatoms including S, O, N and the like, and having 6 to 40 carbon atoms; and aryl having 6 to 40 carbon atoms. In detail, they may be represented by the following Formulas. 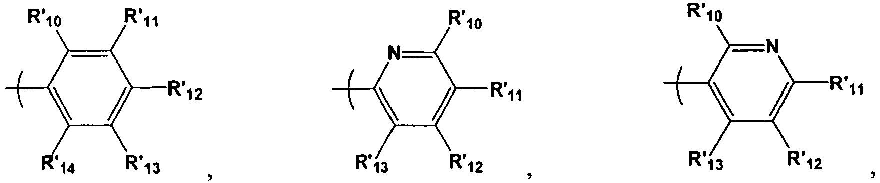 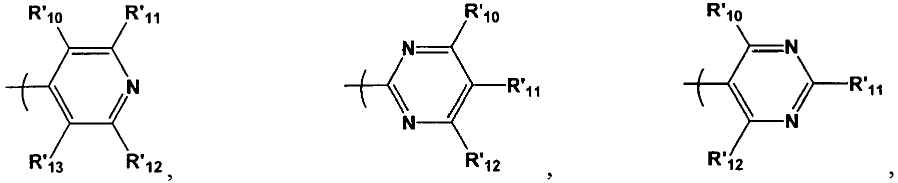 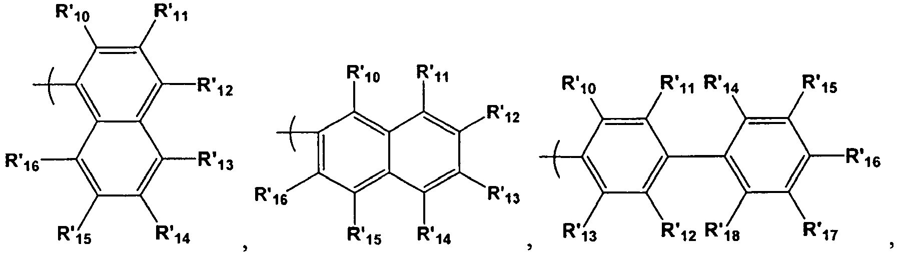 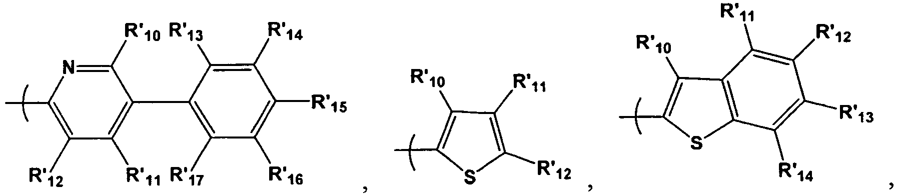 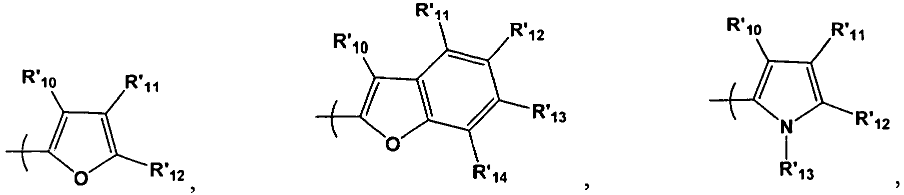 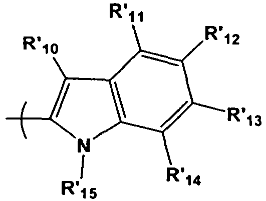 wherein R'₁₀, R'₁₁, R'₁₂, R'₁₃, R'₁₄, R'₁₅, R'₁₆, R'₁₇ and R'₁₈ are each independently selected from the group consisting of hydrogen; halogen; substituted or unsubstituted alkyl having 1 to 20 carbon atoms; substituted or unsubstituted alkoxy having 1 to 20 carbon atoms; substituted or unsubstituted aryloxy having 6 to 30 carbon atoms; and substituted or unsubstituted aryl having 6 to 40 carbon atoms.

The hydrocarbons may be selected from the group consisting of an alkyl group including a methyl group, an ethyl group, a propyl group, an isopropyl group, a butyl group, a tertiary-butyl group, a pentyl group, a hexyl group, and a heptyl group; a cycloalkyl group including a cyclopentyl group and a cyclohexyl group; and an aryl group including a phenyl group, a naphthyl group, an anthracenyl group, a biphenyl group, a pyrenyl group, and a perylenyl group, and in the case of when substituted or unsubstituted alkylene, alkenylene, cyclo alkylene, arylene, aralkylene, and alkynylene are substituted, they may be substituted with the above hydrocarbons.

As the alcohol, alcohol that includes primary alcohol, secondary alcohol, tertiary alcohol and the like, and has straight-chained or cyclic substituent groups such as alkyl group, alkenyl group, aryl group and the like may be used.

For example, examples of the alcohol may include aliphatic primary alcohols including methanol, ethanol, n-propanol, n-butanol, n-hexanol, n-heptanol, n-octanol, n-decanol, n-dodecanol, stearyl alcohol, 2-ethylhexane-1-ol and neopentyl alcohol; aromatic primary alcohols of benzyl alcohol; aliphatic secondary alcohols including isopropyl alcohol, s-butyl alcohol and 1-methylhexane-1-ol; alicyclic secondary alcohols including cyclohexanol and 2-adamanthirol; tertiary alcohols including t-butyl alcohol, 1-adamanthirol, phenol, o-cresol, m-cresol, p-cresol, 3,5-dimethylphenol, α-naphthol and β-naphthol; and polyvalent alcohols including ethylene glycol, propylene glycol, trimethylene glycol, 1,4-butanediol, 1,5-pentanediol, 1,6-hexanediol, 1,7-heptanediol, 1,8-octanediol, pinacol, neopentyl glycol, trimethylol propane, trimethylol ethane, pentaerythritol, dipentaerythritol, sorbitol and polyvinyl alcohol.

The alcohol may be used alone or as a mixture of two or more species. For example, in polyvalent alcohol that has a primary hydroxy group and a secondary hydroxy group, a condensation reaction of a carboxylic acid having a large volume and a primary hydroxy group may be selectively generated, or as a distance between the primary hydroxy group and the secondary hydroxy group is increased, a condensation reaction with the primary hydroxy group may be selectively generated, thus an ester condensate may be chemically selectively generated.

The carboxylic acid that is used in the production method of the ester compound according to the present invention includes compounds that are represented by the following Formulas 8 to 13.

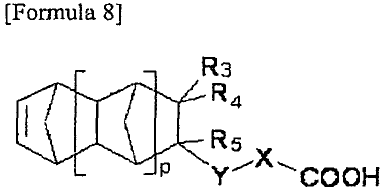

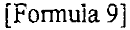

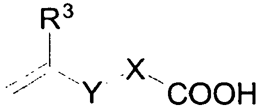

In Formula 8 and 9,
p is an integer in the range of 0 to 4,
Y is a direct bond; or selected from the group consisting of -C(O)-O-, -O-C(O)-, -C(O)N(R₃)-, -OC(O)N(R₃)-, - N(R₃)-, -C(O)-, -SO₂-, -SO₃- and -OSO₂-,
X is selected from the group consisting of a direct bond; -(OCH₂)q- (q is an integer in the range of 0 to 10); substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkynylene having 2 to 20 carbon atoms;
R₃, R₄ and R₅ are each independently selected from the group consisting of hydrogen; halogen; straight- or branched-chained alkyl having 1 to 20 carbon atoms; straight- or branched-chained alkenyl or vinyl having 2 to 20 carbon atoms; cycloalkyl that is unsubstituted or substituted with hydrocarbons and has 5 to 12 carbon atoms; aryl that is unsubstituted or substituted with hydrocarbons and has 6 to 40 carbon atoms; aralkyl that is unsubstituted or substituted with hydrocarbons and has 7 to 15 carbon atoms; and alkynyl having 2 to 20 carbon atoms,

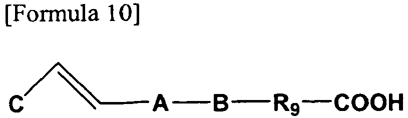

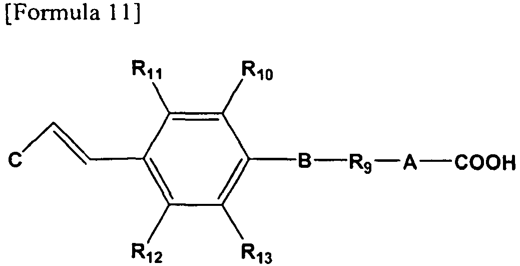

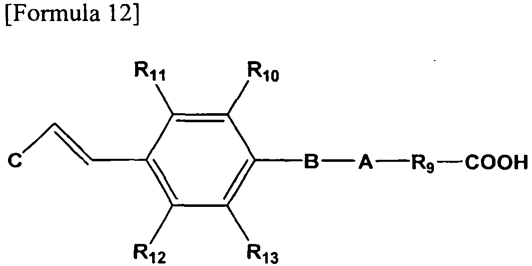

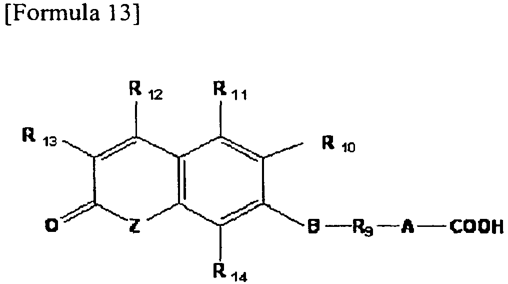

In Formula 10 to 13,
A, B, C, Z, R₉, R₁₀, R₁₁, R₁₂, R₁₃ and R₁₄ are as defined in Formulas 4 to 7.

The hydrocarbons may be selected from the group consisting of an alkyl group including a methyl group, an ethyl group, a propyl group, an isopropyl group, a butyl group, a tertiary-butyl group, a pentyl group, a hexyl group, and a heptyl group; a cycloalkyl group including a cyclopentyl group and a cyclohexyl group; and an aryl group including a phenyl group, a naphthyl group, an anthracenyl group, a biphenyl group, a pyrenyl group, and a perylenyl group, and in the case of when substituted or unsubstituted alkylene, alkenylene, cyclo alkylene, arylene, aralkylene, and alkynylene are substituted, they may be substituted with the above hydrocarbons.

It is preferable that the carboxylic acid used in the esterification reaction is chain type or cyclic type of fatty acids or aromatic acids as monocarboxylic acids or acids having unsaturated bonds or substituent groups to them.

For example, the carboxylic acid compound may include fatty acids including acetic acid, a propionic acid, a n-butyric acid, an isobutyric acid, a n-valeric acid, an isovaleric acid, methylethyl acetic acid, trimethyl acetic acid, a caproic acid, an enantic acid, a caprylic acid, a pelargonic acid, a capric acid, an undercylic acid, a lauric acid, a tridecylic acid, a myricstic acid, a pentadecylic acid, a palmitic acid, a heptadecylic acid, a stearic acid, an acrylic acid, a crotonic acid, an isocrotonic acid, a undecylenic acid, an oleic acid, an elaidic acid, an erucic acid, a brassidic acid, a sorbic acid, a linoleic acid and a linolenic acid; aromatic acids such as benzoic acid; dicarboxylic acids such as a malonic acid, a succinic acid, a glutaric acid, an adipic acid, a pimelic acid, a suberic acid, an azelaic acid, a sebacic acid, a dodecanedioic acid, a fumaric acid, a maleic acid, a mesaconic acid, a citraconic acid, a phthalic acid, a terephthalic acid, an isophthalic acid and a diphenyl ether-4,4'-dicarboxylic acid; tricarboxylic acids including a butane-1,2,4-tricarboxylic acid, a cyclohexane-1,2,3-tricarboxylic acid, a benzene-1,2,4-tricarboxylic acid and a naphthalene-1,2,4-tricarboxylic acid; and tetracarboxylic acids including a butane-1,2,3,4-tetracarboxylic acid, a cyclobutane-1,2,3,4-tetracarboxylic acid, a benzene-1,2,4,5-tetracarboxylic acid, a 3,3',4,4'-benzophenone tetracarboxylic acid and a 3,3',4,4'-diphenylether tetracarboxylic acid.

In the method for producing an ester compound according to the present invention, by using the esterification catalyst composition according to the present invention, alcohol and carboxylic acid compounds may be used in the same mole number, thus efficiently producing an ester compound. In detail, if the monovalent carboxylic acid and alcohol are used, an ester monomer is obtained, and if polyvalent carboxylic acid such as α,ω-aliphatic dicarboxylic acids and polyvalent alcohol such as α,ω-aliphatic diol are used, polyester may be synthesized.

In addition, in the production method of the ester compound, it is preferable that the esterification catalyst composition is added at a ratio of 0.01 to 20 on the basis of a mole number of the compound that is added in a smaller amount among the alcohol and carboxylic acid compounds in the reaction solution. If the amount of the esterification catalyst composition is less than 0.01 mole, there is a problem in that the yield is reduced. If the amount is more than 20 mole, there is a problem in that since reaction byproducts are increased.

In addition, as the alcohol and carboxylic acid that are used in the production method of the ester compound according to the present invention, a ω-hydroxycarboxylic acid that each has independently a hydroxyl group and a carboxy group at both ends in one molecule may be used.

For example, the ω -hydroxycarboxylic acid includes ω-hydroxy undecanoic acid, hydroxy dodecanoic acid, p-hydroxy benzoic acid, m-hydroxy benzoic acid, 6-hydroxynaphthalene-2-carboxylic acid, 4-(p-hydroxyphenoxy) benzoic acid, 3-(p-hydroxyphenoxy) benzoic acid, 4-(m-hydroxyphenoxy) benzoic acid and 3-(m-hydroxyphenoxy) benzoic acid.

The solvent that is used in the production method of the ester compound according to the present invention is not particularly limited, and a polar solvent, a nonpolar solvent or a mixed solvent thereof may be used. The nonpolar solvent is preferable because of easiness of removal of the products by the esterification reaction to the outside of the reaction system. The organic solvent used while the catalyst composition according to the present invention is dissolved therein is preferable because it forms an azeotropic point in conjunction with water.

It is preferable that the total weight of the organic solvent in the above reaction system is in the range of 0.1 to 100 on the basis of a weight of the compound that is added in a smaller amount among the alcohol and carboxylic acid compounds, and it is more preferable that the total weight is in the range of 0.5 to 50.

It is preferable that the nonpolar solvent is selected from the group consisting of heptane, octane, toluene, chlorobenzene, o-xylene, m-xylene, p-xylene, mecithylene, pentamethylbenzene, benzene, ethylbenzene, 1,3,5-triisopropylbenzene, o-dichlorobenzene, 1,2,4-trichlorobenzene and a mixture thereof.

Since the esterification catalyst composition according to the present invention may reduce deterioration in catalyst by moisture adsorption, the esterification reaction using this may be smoothly performed under an atmospheric exposure state. In addition, the esterification reaction may be carried out under a dry inert gas atmosphere generally used, for example, under an argon or nitrogen atmosphere. In addition, the argon atmosphere may be generated by using a method where argon is allowed to slowly flow, and dehydration and deoxygenation atmospheres may be simultaneously accomplished by performing the reaction under the argon atmosphere.

In addition, in the condensation reaction of the monovalent carboxylic acid and monovalent alcohol or the polycondensation reaction of aliphatic polyvalent carboxylic acid and aliphatic polyvalent alcohol, it is preferable that the reaction is carried out under hot reflux at 100 to 250°C for 24 to 72 hours. It is more preferable that the reaction is carried out at 120 to 180°C for 1 to 24 hours.

Meanwhile, in the condensation reaction of the aromatic carboxylic acid and the aromatic alcohol, it is preferable that the reaction is carried out under hot reflux at 120 to 250°C. It is more preferable that the reaction is carried out at 150 to 200°C for 24 to 72 hours.

In the present invention, since the ester compound that is obtained by the above esterification reaction condition does not generate side reactions by using the carboxylic acid and alcohol in almost the same mole number, it is not necessary to perform the purification, thus it is economical and convenient.

After the reaction of ester is finished, in order to repeatedly use the used catalyst, the following treatment may be carried out. The ionic liquid is added to the reaction system, the zirconium compound that is included in the esterification catalyst composition according to the present invention is extracted with the ionic liquid layer by using the ionic liquid, and the ester compound may be obtained from the organic layer. Here, the ionic liquid means a salt that has a property where the salt becomes liquid at room temperature or at a temperature that is close to room temperature. Since it has very high polarity and well dissolves metal salts, it may be suitably used to extract the catalyst used in the production method of the ester compound according to the present invention. The ionic liquid extracting the catalyst is washed with another organic solvent according to the necessity, and may be repeatedly used as a catalyst solution of the esterification reaction while the solvent is not removed under reduced pressure.

The ionic liquid that is used in the production method of the ester compound according to the present invention is not limited, but preferably 1-butyl-3-methylimidasoliumtrifluoromethansulfonimide, 1-ethyl-3-methylimidasoliumtrifluoromethansulfonate, N-alkylpyrydiniumtrifluoromethansulfonateimide, and more preferably N-butylpyrydiniumtrifluoromethansulfonateimide. The use amount of the ionic liquid is in the range of preferably 5 to 20 mℓ and more preferably 10 to 15 mℓ on the basis of 0.5 mmol of the total use amount of the catalyst.

### [Mode for Invention]

Hereinafter, the present invention will be described in detail in light of Examples and Experimental Examples. The present invention may, however, be embodied in many different forms and should not be construed as being limited to the Examples and Experimental Examples set forth herein. Rather, these Examples and Experimental Examples are provided such that this disclosure will be thorough and complete and will fully convey the concept of the present invention to those skilled in the art.

In the following Examples, the solvent such as xylene, toluene and the like was directly used while being not particularly purified.

### Production of the ester compound

### Example 1: Reaction of 5-norbomene-2-methanol and the cinnamic acid - Use of the zirconium carbonate basic hydrate catalyst

5-norbornene-2-methanol (3.72 g, 30 mmol), the cinnamic acid (4.23 g, 33 mmol), and 30 mℓ of xylene were continuously added to the 250 mℓ flask. Zirconium carbonate basic hydrate [3ZrO₂ · CO₂ · (H₂O)_{X}] (1.3 g, 3 mmol, 10 mol%) was added thereto, and azeotropic reflux was performed. The temperature of the heated bath was controlled to 180°C and the reaction was performed for 18 hours. After that, the reaction was finished after the GC check, and it was cooled to normal temperature. Thereafter, 30 mℓ of ethyl acetic acid was added thereto. After the undissolved solid compound is precipitated, 30 mℓ of 1 M (mol/L) dilute hydrochloric acid solution was added thereto, and the washing was performed. This process was performed twice, and 30 mℓ of the saturated aq.NaHCO₃ solution was added thereto and washed. This process was performed twice, and MgSO₄ was added to the separated organic solution to remove a small amount of water. After this was filtered by using MgSO₄, the solvent was removed under reduced pressure, and the purification process was carried out to obtain 6.95 g of cinnamic norbornene methyl ester (NB-Cin-H compound) that was the light yellow liquid compound (molecular weight = 284.35, yield 90.7%).

### Example 4: Reaction of benzyl alcohol and the 4-phenyl butyric acid - Use of the zirconium carbonate basic hydrate catalyst

Benzyl alcohol (1.62 g, 15 mmol), 4-phenyl butyric acid (2.46 g, 15 mmol) and 25 mℓ of toluene were continuously added to the 250 mℓ flask. Zirconium carbonate basic hydrate [3ZrO₂ · CO₂ · (H₂O)_{X}] (0.0648 g, 0.15 mmol, 1 mol%) was added thereto, and azeotropic reflux was performed. The temperature of the heated bath was controlled to 150°C and the reaction was performed for 18 hours. After that, the reaction was finished after the GC check, and it was cooled to normal temperature. Thereafter, 30 mℓ of ethyl acetic acid was added thereto. After the undissolved solid compound is precipitated, 30 mℓ of 1 M (mol/L) dilute hydrochloric acid solution was added thereto, and the washing was performed. This process was performed twice, and 30 mℓ of the saturated aq.NaHCO₃ solution was added thereto and washed. This process was performed twice, and MgSO₄ was added to the separated organic solution to remove a small amount of water. After water was removed, this was filtered by using MgSO₄, and the solvent was removed under reduced pressure. The purification process was carried out to obtain 2.8 g of the colorless liquid compound (molecular weight = 189.24, yield 98.6%).

### [Industrial Applicability]

According to an esterification catalyst composition and a method for producing an ester compound using the same according to the present invention, since the solubility to a nonpolar solvent is excellent and the activity of the reaction is excellent in an atmosphere exposure state, they are very easily applied to a mass production process.

## Claims

1. An esterification catalyst composition comprising zirconium carbonate basic hydrate [3ZrO₂.CO₂.(H₂O)ₓ], wherein x is an integer in the range of 1 to 12.

2. The esterification catalyst composition as set forth in claim 1, wherein the esterification catalyst composition is carried in a particulate support including one or more selected from the group consisting of silica, titania, silica/cromia, silica/cromia/titania, silica/alumina, aluminium phosphate gel, silanated silica, silica hydro gel, montmorilonite clay and zeolite.

3. A method for producing an ester compound, the method comprising the steps of:
esterifying alcohol and carboxylic acid compounds by using the esterification catalyst composition according claim 1,
wherein the alcohol includes one or more selected from the group consisting of compounds that are represented by the following Formulas 2 to 7: 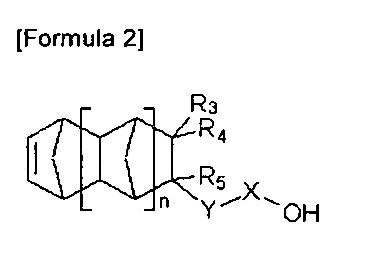 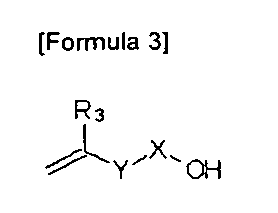
wherein n is an integer in the range of 0 to 4,
Y is a direct bond, or is selected from the group consisting of -C(O)-O-, - O-C(O)-, -C(O)N(R₃)-, -OC(O)N(R₃)-, -N(R₃)-, -C(O)-, -SO₂-, -SO₃- and -OSO₂-,
X is selected from the group consisting of a direct bond; -(OCH₂) m- (m is an integer in the range of 0 to 10); substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkenylene having 2 to 20 carbon atoms;
R₃, R₄ and R₅ are each independently selected from the group consisting of hydrogen; halogen; straight- or branched-chained alkyl having 1 to 20 carbon atoms; straight- or branched-chained vinyl or alkenyl having 2 to 20 carbon atoms; alkynyl having 2 to 20 carbon atoms; cycloalkyl that is unsubstituted or substituted with hydrocarbons and has 5 to 12 carbon atoms; aryl that is unsubstituted or substituted with hydrocarbons and has 6 to 40 carbon atoms; and aralkyl that is unsubstituted or substituted with hydrocarbons and has 7 to 15 carbon atoms, 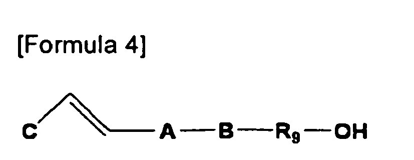 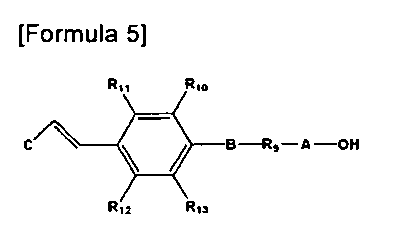 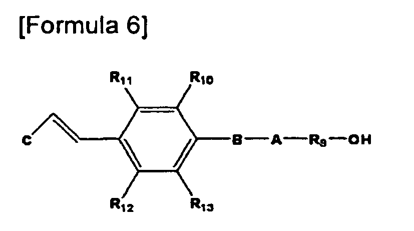 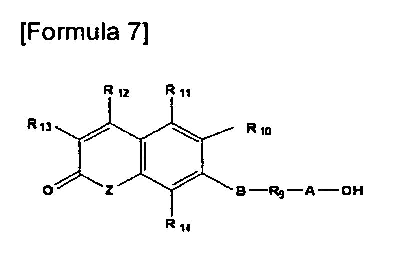
wherein A is a direct bond; or selected from the group consisting of substituted or unsubstituted alkylene having 1 to 20 carbon atoms; carbonyl [-C(O)-]; carboxy [-C(O)O- or -OC(O)-]; and substituted or unsubstituted arylene having 6 to 40 carbon atoms,
B is a direct bond; oxygen, sulfur or -NH-,
Z is oxygen or sulfur,
R9 is a direct bond; or selected from the group consisting of substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkynylene having 2 to 20 carbon atoms,
R₁₀, R₁₁, R₁₂, R₁₃ and R₁₄ are each independently selected from the group consisting of substituted or unsubstituted alkyl having 1 to 20 carbon atoms; substituted or unsubstituted alkoxy having 1 to 20 carbon atoms; substituted or unsubstituted aryloxy having 6 to 30 carbon atoms; and substituted or unsubstituted aryl having 6 to 40 carbon atoms, and
C is a compound that includes hetero aryl including Groups 14, 15, or 16 hetero atoms and having 6 to 40 carbon atoms; or aryl having 6 to 40 carbon atoms, and
wherein the carboxylic acid compound includes one or more selected from the group consisting of compounds that are represented by the following Formulas 8 to 13: 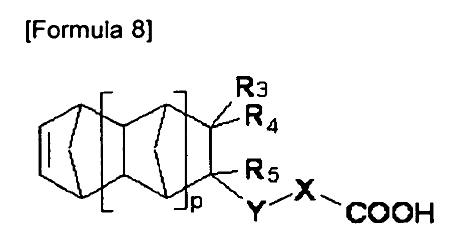 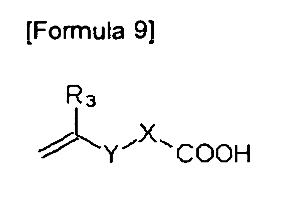
wherein
p is an integer in the range of 0 to 4,
Y is a direct bond; or selected from the group consisting of -C(O)-O-, -O- C(O)-, -C(O)N(R₃)-, -OC(O)N(R₃)-, -N(R₃)-, -C(O)-, -SO₂-, -SO₃- and -OSO₂-,
X is selected from the group consisting of a direct bond; -(OCH₂)q- (q is an integer in the range of 0 to 10); substituted or unsubstituted alkylene having 1 to 20 carbon atoms; substituted or unsubstituted alkenylene having 2 to 20 carbon atoms; substituted or unsubstituted cycloalkylene having 5 to 12 carbon atoms; substituted or unsubstituted arylene having 6 to 40 carbon atoms; substituted or unsubstituted aralkylene having 7 to 15 carbon atoms; and substituted or unsubstituted alkynylene having 2 to 20 carbon atoms;
R₃, R₄ and R₅ are each independently selected from the group consisting of hydrogen; halogen; straight- or branched-chained alkyl having 1 to 20 carbon atoms; straight- or branched-chained vinyl or alkenyl having 2 to 20 carbon atoms; cycloalkyl that is substituted or unsubstituted with hydrocarbons and has 5 to 12 carbon atoms; aryl that is substituted or unsubstituted with hydrocarbons and has 6 to 40 carbon atoms; aralkyl that is substituted or unsubstituted with hydrocarbons and has 7 to 15 carbon atoms; and alkynyl having 2 to 20 carbon atoms, 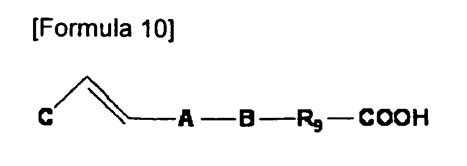 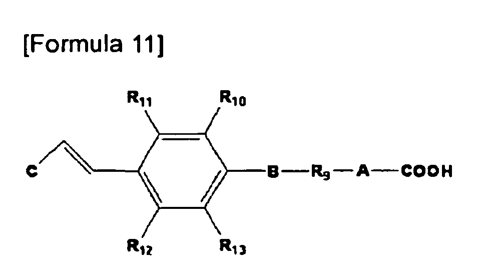 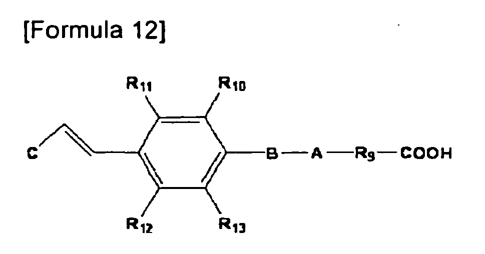 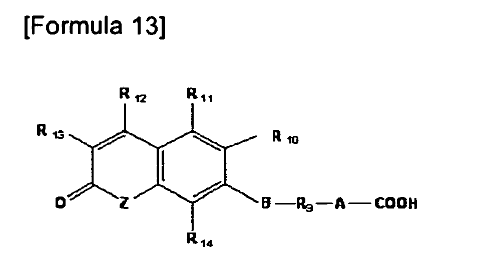
wherein A, B, C, Z, R₉, R₁₀ R₁₁, R₁₂, R₁₃ and R₁₄ are as defined in Formulas 4 to 7.

4. The method for producing an ester compound as set forth in claim 3,
wherein the esterification catalyst composition is used at a ratio of 0.01 to 20 on the basis of a mole number of the compound that is added in a smaller amount among the alcohol and carboxylic acid compounds.

5. The method for producing an ester compound as set forth in claim 3, wherein C of Formulas 4 to 6 is selected from the group consisting of the following 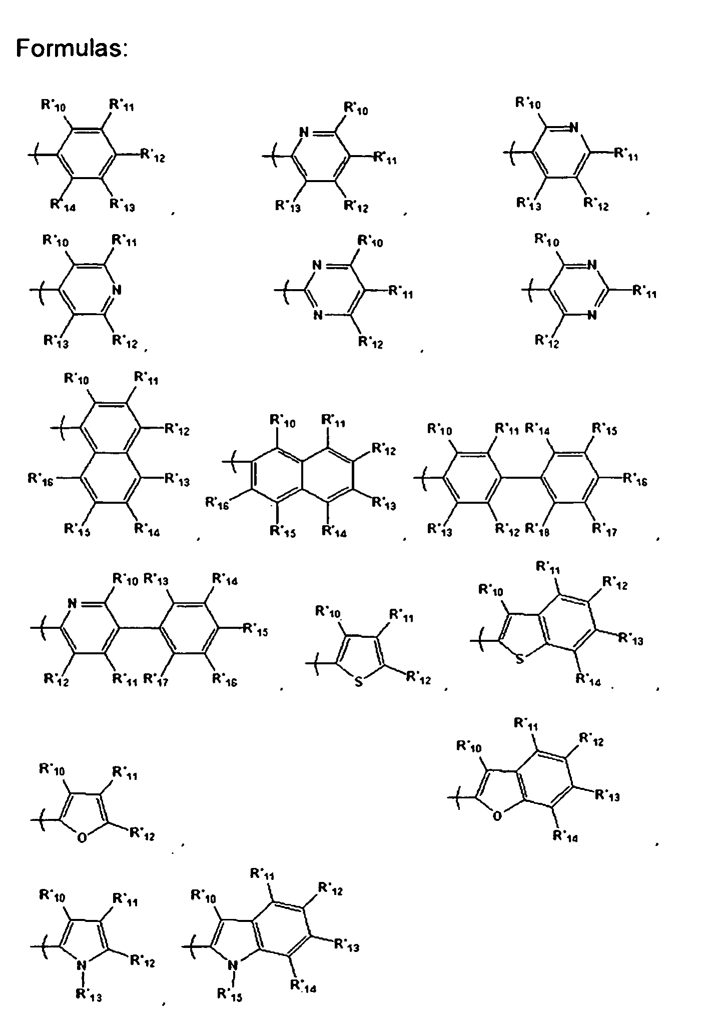 wherein R'₁₀ R'₁₁, R'₁₂, R'₁₃, R'₁₅, R'₁₆, R'₁₇ and R'₁₈ are each independently selected from the group consisting of hydrogen; halogen; substituted or unsubstituted alkyl having 1 to 20 carbon atoms; substituted or unsubstituted alkoxy having 1 to 20 carbon atoms; substituted or unsubstituted aryloxy having 6 to 30 carbon atoms; and substituted or unsubstituted aryl having 6 to 40 carbon atoms.

6. The method for producing an ester compound as set forth in claim 3, wherein the alcohol includes one or more selected from the group consisting of aliphatic primary alcohols including methanol, ethanol, n-propanol, n-butanol, n-hexanol, n-heptanol, n-octanol, n-decanol, n-dodecanol, stearyl alcohol, 2-ethylhexane-1-ol and neopentyl alcohol; aromatic primary alcohols of benzyl alcohol; aliphatic secondary alcohols including isopropyl alcohol, s-butyl alcohol and 1-methylhexane-1-ol; alicyclic secondary alcohols including cyclohexanol and 2-adamanthirol; tertiary alcohols including t-butyl alcohol, 1-adamanthirol, phenol, o-cresol, m-cresol, p-cresol, 3,5-dimethylphenol, α-naphthol and β-naphthol; and polyvalent alcohols including ethylene glycol, propylene glycol, trimethylene glycol, 1,4-butanediol, 1,5-pentanediol, 1,6-hexanediol, 1,7-heptanediol, 1,8-octanediol, pinacol, neopentyl glycol, trimethylol propane, trimethylol ethane, pentaerythritol, dipentaerythritol, sorbitol and polyvinyl alcohol.

7. The method for producing an ester compound as set forth in claim 3, wherein the carboxylic acid compound includes one or more selected from the group consisting of fatty acids including an acetic acid, a propionic acid, a n-butyric acid, an isobutyric acid, a n-valeric acid, an isovaleric acid, methylethyl acetic acid, trimethyl acetic acid, a caproic acid, an enantic acid, a caprylic acid, a pelargonic acid, a capric acid, an undercylic acid, a lauric acid, a tridecylic acid, a myricstic acid, a pentadecylic acid, a palmitic acid, a heptadecylic acid, a stearic acid, an acrylic acid, a crotonic acid, an isocrotonic acid, a undecylenic acid, an oleic acid, an elaidic acid, an erucic acid, a brassidic acid, a sorbic acid, a linoleic acid and a linolenic acid; aromatic acids such as benzoic acid; dicarboxylic acids such as a malonic acid, a succinic acid, a glutaric acid, an adipic acid, a pimelic acid, a suberic acid, an azelaic acid, a sebacic acid, a dodecanedioic acid, a fumaric acid, a maleic acid, a mesaconic acid, a citraconic acid, a phthalic acid, a terephthalic acid, an isophthalic acid and a diphenyl ether-4,4' -di carboxylic acid; tricarboxylic acids including a butane-1,2,4-tricarboxylic acid, a cyclohexane-1,2,3-tricarboxylic acid, a benzene-1,2,4-tricarboxylic acid and a naphthalene-1,2,4-tricarboxylic acid; and tetracarboxylic acids including a butane-1,2,3,4-tetracarboxylic acid, a cyclobutane-1,2,3,4-tetracarboxylic acid, a benzene-1,2,4,5-tetracarboxylic acid, a 3,3' ,4,4'-benzophenone tetracarboxylic acid and a 3,3' ,4,4' -diphenylether tetracarboxylic acid.

8. A method for producing an ester compound, the method comprising the steps of:
esterifying a w-hydroxycarboxylic acid that each has independently a hydroxyl group and a carboxy group at both ends in one molecule using the esterification catalyst composition according to claim 1.

9. The method for producing an ester compound as set forth in claim 8, wherein the w-hydroxycarboxylic acid includes w-hydroxy undecanoic acid, hydroxy dodecanoic acid, p-hydroxy benzoic acid, m-hydroxy benzoic acid, 6-hydroxynaphthalene-2-carboxylic acid, 4-(p-hydroxyphenoxy)benzoic acid, 3-(p-hydroxyphenoxy)benzoic acid, 4-(m-hydroxyphenoxy)benzoic acid and 3-(m-hydroxyphenoxy)benzoic acid.

## Patentansprüche

1. Katalysatorzusammensetzung zur Veresterung umfassend basisches Zirkoniumcarbonathydrat [3ZrO₂.CO₂.(H₂O)ₓ], wobei x eine ganze Zahl in dem Bereich von 1 bis 12 ist.

2. Katalysatorzusammensetzung zur Veresterung nach Anspruch 1, wobei die Katalysatorzusammensetzung zur Veresterung in einem partikelförmigen Träger, einschließend einen oder mehrere ausgewählt aus der Gruppe bestehend aus Siliziumdioxid, Titandioxid, Siliziumdioxid/Dichromoxid, Siliziumdioxid/ Dichromoxid/Titandioxid, Siliziumdioxid/Aluminiumoxid, Aluminiumphosphatgel, silanisiertem Siliziumoxid, Siliziumdioxidhydrogel, Montmorilonitton und Zeolith, getragen wird.

3. Verfahren zum Herstellen einer Esterverbindung, wobei das Verfahren die Schritte umfasst:
Verestern von Alkohol- und Carbonsäureverbindungen durch Verwendung der Katalysatorzusammensetzung zur Veresterung nach Anspruch 1,
wobei der Alkohol einen oder mehrere ausgewählt aus der Gruppe bestehend aus Verbindungen, welche durch die folgenden Formeln 2 bis 7 dargestellt sind, einschließt: 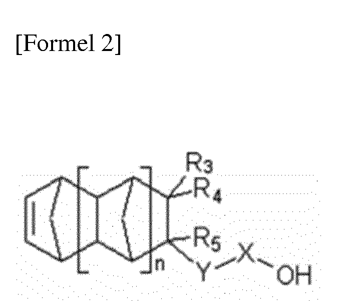 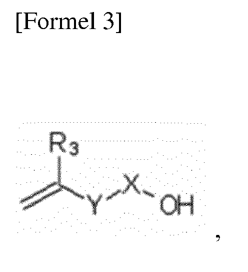
wobei n eine ganze Zahl in dem Bereich von 0 bis 4 ist,
Y eine direkte Bindung ist, oder ausgewählt ist aus der Gruppe bestehend aus -C(O)-O-, -O-C(O)-, -C(O)N(R₃)-, -OC(O)N(R₃)-, -N(R₃)-, -C(O)-, -SO₂-, -SO₃- und -OSO₂-,
X ausgewählt ist aus der Gruppe bestehend aus einer direkten Bindung; -(OCH₂)m- (m ist eine ganze Zahl in dem Bereich von 0 bis 10); substituiertem oder unsubstituiertem Alkylen mit 1 bis 20 Kohlenstoffatomen; substituiertem oder unsubstituiertem Alkenylen mit 2 bis 20 Kohlenstoffatomen; substituiertem oder unsubstituiertem Cycloalkylen mit 5 bis 12 Kohlenstoffatomen; substituiertem oder unsubstituiertem Arylen mit 6 bis 40 Kohlenstoffatomen; substituiertem oder unsubstituiertem Aralkylen mit 7 bis 15 Kohlenstoffatomen; und substituiertem oder unsubstituiertem Alkinylen mit 2 bis 20 Kohlenstoffatomen;
R₃, R₄ und R₅ jeweils unabhängig ausgewählt sind aus der Gruppe bestehend aus Wasserstoff; Halogen; gerad- oder verzweigtkettigem Alkyl mit 1 bis 20 Kohlenstoffatomen; gerad- oder verzweigtkettigem Vinyl oder Alkenyl mit 2 bis 20 Kohlenstoffatomen; Alkinyl mit 2 bis 20 Kohlenstoffatomen; Cycloalkyl, welches unsubstituiert oder mit Kohlenwasserstoffen substituiert ist und 5 bis 12 Kohlenstoffatome hat; Aryl, welches unsubstituiert oder mit Kohlenwasserstoffen substituiert ist und 6 bis 40 Kohlenstoffatome hat; und Aralkyl, welches unsubstituiert oder mit Kohlenwasserstoffen substituiert ist und 7 bis 15 Kohlenstoffatome hat, 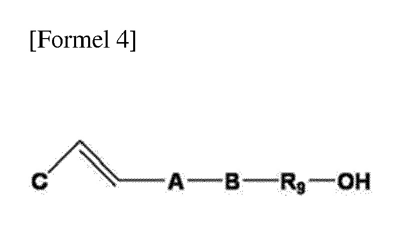 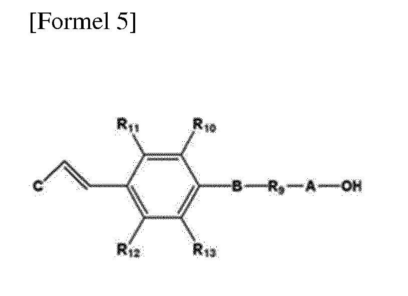 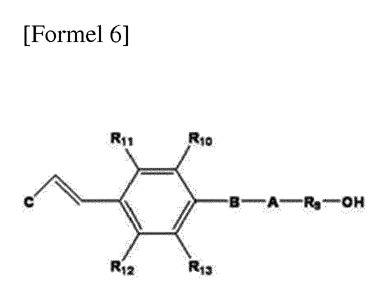 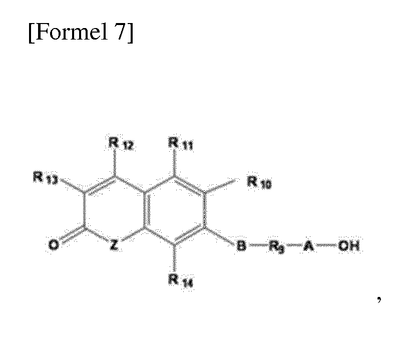
wobei A eine direkte Bindung ist; oder ausgewählt ist aus der Gruppe bestehend aus substituiertem oder unsubstituiertem Alkylen mit 1 bis 20 Kohlenstoffatomen; Carbonyl [-C(O)-]; Carboxy [-C(O)O- oder -OC(O)-]; und substituiertem oder unsubstituiertem Arylen mit 6 bis 40 Kohlenstoffatomen ist,
B eine direkte Bindung; Sauerstoff, Schwefel oder -NH- ist,
Z Sauerstoff oder Schwefel ist,
R9 eine direkte Bindung ist; oder ausgewählt ist aus der Gruppe bestehend aus substituiertem oder unsubstituiertem Alkylen mit 1 bis 20 Kohlenstoffatomen; substituiertem oder unsubstituiertem Alkenylen mit 2 bis 20 Kohlenstoffatomen; substituiertem oder unsubstituiertem Cycloalkylen mit 5 bis 12 Kohlenstoffatomen; substituiertem oder unsubstituiertem Arylen mit 6 bis 40 Kohlenstoffatomen; substituiertem oder unsubstituiertem Aralkylen mit 7 bis 15 Kohlenstoffatomen; und substituiertem oder unsubstituiertem Alkinylen mit 2 bis 20 Kohlenstoffatomen,
R₁₀, R₁₁, R₁₂, R₁₃ und R₁₄ jeweils unabhängig ausgewählt sind aus der Gruppe bestehend aus substituiertem oder unsubstituiertem Alkyl mit 1 bis 20 Kohlenstoffatomen; substituiertem oder unsubstituiertem Alkoxy mit 1 bis 20 Kohlenstoffatomen; substituiertem oder unsubstituiertem Aryloxy mit 6 bis 30 Kohlenstoffatomen; und substituiertem oder unsubstituiertem Aryl mit 6 bis 40 Kohlenstoffatomen, und
C eine Verbindung ist, welche Heteroaryl enthält, einschließend Heteroatome der Gruppe 14, 15 oder 16 und mit 6 bis 40 Kohlenstoffatomen; oder Aryl mit 6 bis 40 Kohlenstoffatomen ist, und
wobei die Carbonsäureverbindung eine oder mehrere ausgewählt aus der Gruppe bestehend aus Verbindungen, welche durch die folgenden Formeln 8 bis 13 dargestellt sind, einschließt: 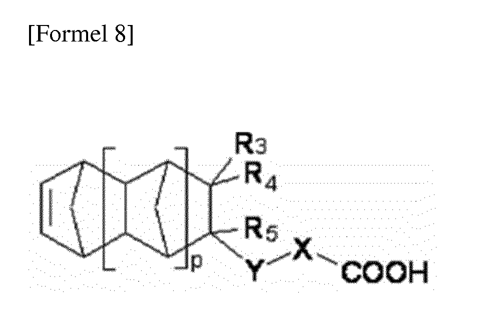 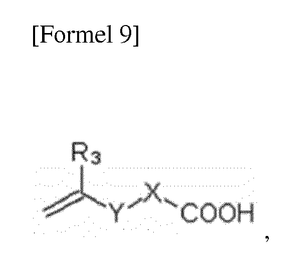
wobei
p eine ganze Zahl in dem Bereich von 0 bis 4 ist,
Y eine direkte Bindung ist; oder ausgewählt ist aus der Gruppe bestehend aus -C(O)-O-, -O-C(O)-, -C(O)N(R₃)-, -OC(O)N(R₃)-, -N(R₃)-, -C(O)-, -SO₂-, -SO₃- und -OSO₂-,
X ausgewählt ist aus der Gruppe bestehend aus einer direkten Bindung; -(OCH₂)q- (q ist eine ganze Zahl in dem Bereich von 0 bis 10); substituiertem oder unsubstituiertem Alkylen mit 1 bis 20 Kohlenstoffatomen; substituiertem oder unsubstituiertem Alkenylen mit 2 bis 20 Kohlenstoffatomen; substituiertem oder unsubstituiertem Cycloalkylen mit 5 bis 12 Kohlenstoffatomen; substituiertem oder unsubstituiertem Arylen mit 6 bis 40 Kohlenstoffatomen; substituiertem oder unsubstituiertem Aralkylen mit 7 bis 15 Kohlenstoffatomen; und substituiertem oder unsubstituiertem Alkinylen mit 2 bis 20 Kohlenstoffatomen;
R₃, R₄ und R₅ jeweils unabhängig ausgewählt sind aus der Gruppe bestehend aus Wasserstoff; Halogen; gerad- oder verzweigtkettigem Alkyl mit 1 bis 20 Kohlenstoffatomen; gerad- oder verzweigtkettigem Vinyl oder Alkenyl mit 2 bis 20 Kohlenstoffatomen; Cycloalkyl, welches unsubstituiert oder mit Kohlenwasserstoffen substituiert ist und 5 bis 12 Kohlenstoffatome hat; Aryl, welches unsubstituiert oder mit Kohlenwasserstoffen substituiert ist und 6 bis 40 Kohlenstoffatome hat; Aralkyl, welches unsubstituiert oder mit Kohlenstoffatomen substituiert ist und 7 bis 15 Kohlenstoffatome hat; und Alkinyl, welches 2 bis 20 Kohlenstoffatome hat, 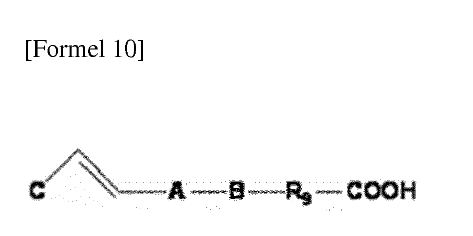 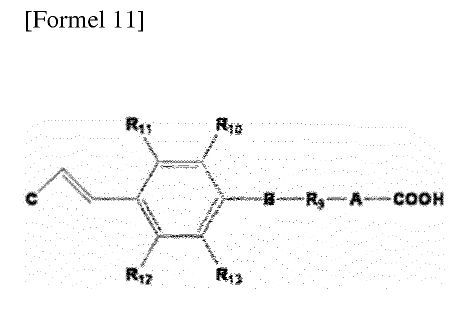 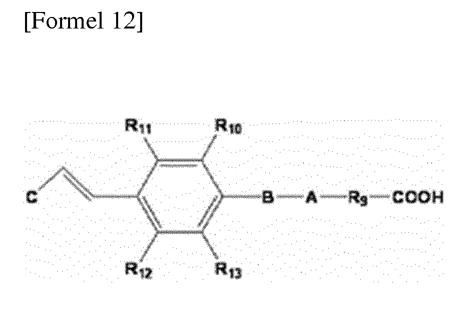 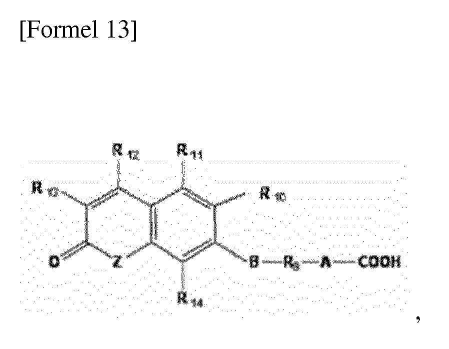
wobei A, B, C, Z, R₉, R₁₀, R₁₁, R₁₂, R₁₃ und R₁₄ wie in den Formeln 4 bis 7 definiert sind.

4. Verfahren zum Herstellen einer Esterverbindung nach Anspruch 3, wobei die Katalysatorzusammensetzung zur Veresterung in einem Verhältnis von 0,01 bis 20 auf der Basis einer Molzahl der Verbindung, welche in einer kleineren Menge neben den Alkohol- und Carbonsäureverbindungen zugegeben wird, verwendet wird.

5. Verfahren zum Herstellen einer Esterverbindung nach Anspruch 3, wobei C aus Formeln 4 bis 6 ausgewählt ist aus der Gruppe bestehend aus den folgenden Formeln: 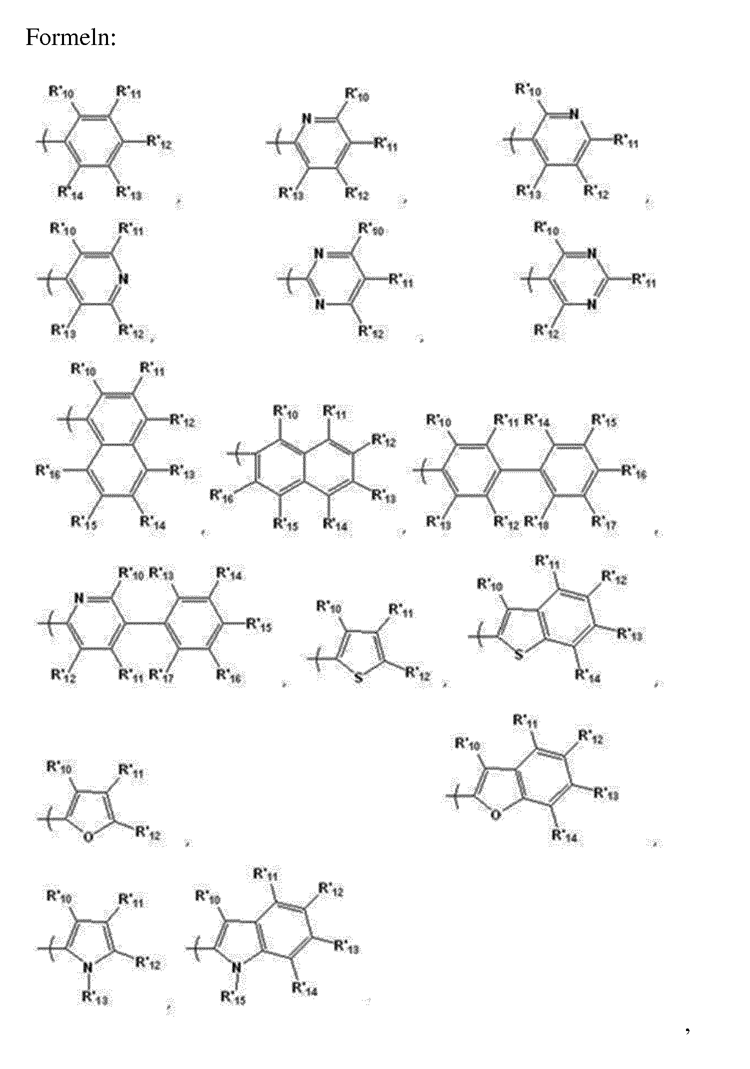 wobei R'₁₀, R'₁₁, R'₁₂, R'₁₃, R'₁₅, R'₁₆, R'₁₇ und R'₁₈ jeweils unabhängig ausgewählt sind aus der Gruppe bestehend aus Wasserstoff; Halogen; substituiertem oder unsubstituiertem Alkyl mit 1 bis 20 Kohlenstoffatomen; substituiertem oder unsubstituiertem Alkoxy mit 1 bis 20 Kohlenstoffatomen; substituiertem oder unsubstituiertem Aryloxy mit 6 bis 30 Kohlenstoffatomen; und substituiertem oder unsubstituiertem Aryl mit 6 bis 40 Kohlenstoffatomen.

6. Verfahren zum Herstellen einer Esterverbindung nach Anspruch 3, wobei der Alkohol einen oder mehrere ausgewählt aus der Gruppe bestehend aus aliphatischen primären Alkoholen einschließend Methanol, Ethanol, n-Propanol, n-Butanol, n-Hexanol, n-Heptanol, n-Octanol, n-Decanol, n-Dodecanol, Sterylalkohol, 2-Ethylhexan-1-ol und Neopentylalkohol; aromatischen primären Alkoholen aus Benzylalkohol; aliphatischen sekundären Alkoholen einschließend Isopropylalkohol, s-Butylalkohol und 1-Methylhexan-1-ol; alizyklischen sekundären Alkoholen einschließend Cyclohexanol und 2-Adamanthirol; tertiären Alkoholen einschließend t-Butylalkohol, 1-Adamanthirol, Phenol, o-Cresol, m-Cresol, p-Cresol, 3,5-Dimethylphenol, α-Naphthol und β-Naphthol; und polyvalenten Alkoholen einschließend Ethylenglykol, Propylenglykol, Trimethylenglykol, 1,4-Butandiol, 1,5-Pentandiol, 1,6-Hexandiol, 1,7-Heptandiol, 1,8-Octandiol, Pinacol, Neopentylglykol, Trimethylolpropan, Trimethylolethan, Pentaerythritol, Dipentaerythritol, Sorbitol und Polyvinylalkohol einschließt.

7. Verfahren zum Herstellen einer Esterverbindung nach Anspruch 3, wobei die Carbonsäureverbindung einen oder mehrere ausgewählt aus der Gruppe bestehend aus Fettsäuren einschließend eine Essigsäure, eine Propionsäure, eine n-Butylsäure, eine Isobutylsäure, eine n-Valerinsäure, eine Isovalerinsäure, Methylethylessigsäure, Trimethylessigsäure, eine Capronsäure, eine Enanthsäure, eine Caprylsäure, eine Pelargonsäure, eine Caprinsäure, eine unterzyklische Säure, eine Laurinsäure, eine Tridecansäure, eine Myristinsäure, eine pentadezyklische Säure, eine Palmatinsäure, eine heptadezyklische Säure, eine Stearinsäure, eine Acrylsäure, eine Crotonsäure, eine Isocrotonsäure, eine Undecylensäure, eine Ölsäure, eine Elaidinsäure, eine Erucasäure, eine Brassidinsäure, eine Sorbinsäure, eine Linolsäure und eine Linolensäure; aromatischen Säuren wie Benzoesäure; Dicarbonsäuren wie eine Malonsäure, eine Succinsäure, eine Glutarsäure, eine Adipinsäure, eine Pimelinsäure, eine Korksäure, eine Azelainsäure, eine Sebacinsäure, eine Dodecandisäure, eine Fumarsäure, eine Maleinsäure, eine Mesaconsäure, eine Citraconsäure, eine Phthalsäure, eine Terephthalsäure, eine Isophthalsäure und eine Diphenylether-4,4'-dicarbonsäure; Tricarbonsäuren einschließend eine Butan-1,2,4-tricarbonsäure, eine Cyclohexan-1,2,3-tricarbonsäure, eine Benzen-1,2,4-tricarbonsäure und eine Naphthalin-1,2,4-tricarbonsäure; und Tetracarbonsäuren einschließend eine Butan-1,2,3,4-tetracarbonsäure, eine Cyclobutan-1,2,3,4-tetracarbonsäure, eine Benzen-1,2,4,5-tetracarbonsäure, eine 3,3'-, 4,4'-Benzophenontetracarbonsäure und eine 3,3'-, 4,4'-Diphenylethertetracarbonsäure einschließt.

8. Verfahren zum Herstellen einer Esterverbindung, umfassend die Schritte:
Verestern einer ω-Hydroxycarbonsäure, welche jeweils unabhängig eine Hydroxylgruppe und eine Carboxygruppe an beiden Enden in einem Molekül, verwendend die Katalysatorzusammensetzung zur Veresterung nach Anspruch 1, hat.

9. Verfahren zum Herstellen einer Esterverbindung nach Anspruch 8, wobei die ω-Hydroxycarbonsäure ω-Hydroxyundecansäure, Hydroxydodecansäure, p-Hydroxybenzoesäure, m-Hydroxybenzoesäure, 6-Hydroxynaphthalin-2-carbonsäure, 4-(p-Hydroxyphenoxy)benzoesäure, 3-(p-Hydroxyphenoxy)benzoesäure, 4-(m-Hydroxyphenoxy)benzoesäure und 3-(m-Hydroxyphenoxy)benzoesäure einschließt.

## Revendications

1. Composition catalysante d'estérification comprenant de l'hydrate basique de carbonate de zirconium [3ZrO₂.CO₂.(H₂O)ₓ], dans laquelle x est un entier compris entre 1 et 12.

2. Composition catalysante d'estérification selon le mode opératoire de la revendication 1, dans laquelle la composition catalysante d'estérification est portée dans un support particulaire comprenant un ou plusieurs éléments sélectionnés parmi le groupe constitué de silice, titane, silice/chrome, silice/chrome/titane, silice/alumine, gel de phosphate d'aluminium, silice silylée, gel d'hydro-silice, argile montmorilonite et zéolite.

3. Procédé de production d'un composé d'ester, le procédé comprenant les étapes consistant à :
estérifier des composés d'alcool et d'acide carboxylique en utilisant la composition catalysante d'estérification selon la revendication 1,
dans lequel l'alcool comprend un ou plusieurs éléments sélectionnés parmi le groupe constitué des composés qui sont représentés par les Formules 2 à 7 suivantes : 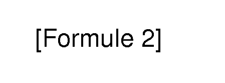 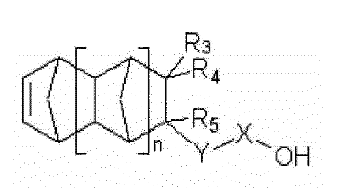 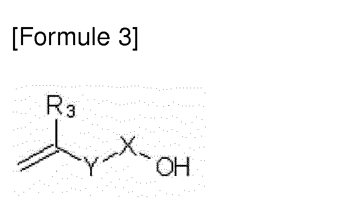
dans lesquelles n représente un entier compris entre 0 et 4,
Y est une liaison directe, ou est sélectionné parmi le groupe constitué de - C(O)-O-, -O-C(O)-, -C(O)N(R₃)-, -OC(O)N(R₃)-, -N(R₃)-, - C(O)-, -SO₂-, -SO₃- et -OSO₂-,
X est sélectionné parmi le groupe constitué d'une liaison directe ; -(OCH₂) m- (m représente un entier compris entre 0 et 10) ; d'alkylène substitué ou non substitué portant 1 à 20 atomes de carbone ; d'alkénylène substitué ou non substitué portant 2 à 20 atomes de carbone ; de cycloalkylène substitué ou non substitué portant 5 à 12 atomes de carbone ; d'arylène substitué ou non substitué portant 6 à 40 atomes de carbone ; d'aralkylène substitué ou non substitué portant 7 à 15 atomes de carbone ; d'alkynylène substitué ou non substitué portant 2 à 20 atomes de carbone ;
R₃, R₄ et R₅ sont chacun indépendamment sélectionnés parmi le groupe constitué d'hydrogène ; d'halogène ; d'alkyle linéaire ou ramifié portant 1 à 20 atomes de carbone ; de vinyle ou d'alkényle linéaire ou ramifié portant 2 à 20 atomes de carbone ; d'alkynyle portant 2 à 20 atomes de carbone ; de cycloalkyle non substitué ou substitué par des hydrocarbures et portant 5 à 12 atomes de carbone ; d'aryle non substitué ou substitué par des hydrocarbures et portant 6 à 40 atomes de carbone ; et d'aralkyle non substitué ou substitué par des hydrocarbures et portant 7 à 15 atomes de carbone, 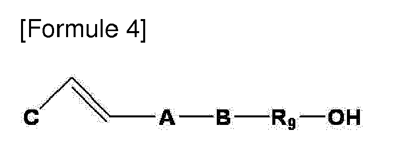 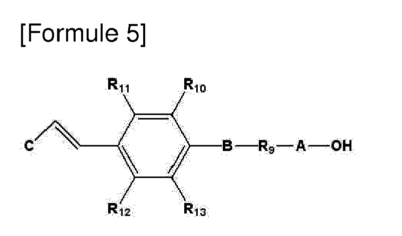 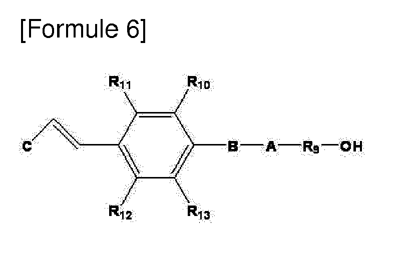 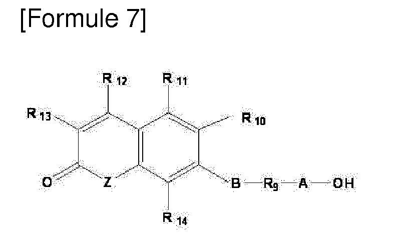
dans lesquelles A représente une liaison directe ; ou est sélectionné parmi le groupe constitué d'alkylène substitué ou non substitué portant 1 à 20 atomes de carbone ; de carbonyle [-C(O)-] ; de carboxy [-C(O)O- ou -OC(O)-] ; et d'arylène substitué ou non substitué portant 6 à 40 atomes de carbone,
B représente une liaison directe ; oxygène, soufre ou -NH-,
Z représente oxygène ou soufre,
R9 représente une liaison directe ; ou est sélectionné parmi le groupe constitué d'alkylène substitué ou non substitué portant 1 à 20 atomes de carbone ; d'alkénylène substitué ou non substitué portant 2 à 20 atomes de carbone ; de cycloalkylène substitué ou non substitué portant 5 à 12 atomes de carbone ; d'arylène substitué ou non substitué portant 6 à 40 atomes de carbone ; d'aralkylène substitué ou non substitué portant 7 à 15 atomes de carbone ; et d'alkynylène substitué ou non substitué portant 2 à 20 atomes de carbone,
R₁₀, R₁₁, R₁₂, R₁₃ et R₁₄ sont chacun indépendamment sélectionnés parmi le groupe constitué d'alkyle substitué ou non substitué portant 1 à 20 atomes de carbone ; d'alcoxy substitué ou non substitué portant 1 à 20 atomes de carbone ; d'aryloxy substitué ou non substitué portant 6 à 30 atomes de carbone ; et d'aryle substitué ou non substitué portant 6 à 40 atomes de carbone, et
C représente un composé contenant de l'hétéroaryle, y compris des hétéroatomes des Groupes 14, 15 ou 16 et portant 6 à 40 atomes de carbone ; ou de l'aryle portant 6 à 40 atomes de carbone, et
dans le composé d'acide carboxylique comprend un ou plusieurs éléments sélectionnés parmi le groupe constitué des composés représentés par les Formules 8 à 13 suivantes : 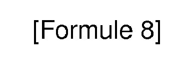 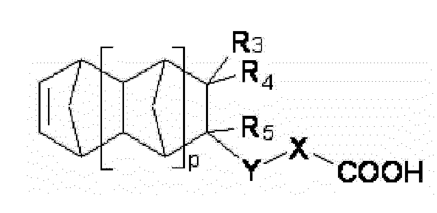 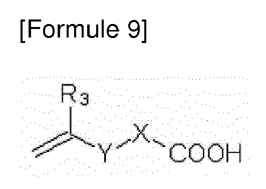
dans lesquelles
p est un entier compris entre 0 à 4,
Y représente une liaison directe ; ou est sélectionné parmi le groupe constitué de -C(O)-O-, -O-C(O)-, -C(O)N(R₃)-, -OC(O)N(R₃)-, -N(R₃)-, -C(O)-, -SO₂-, -SO₃- et -OSO₂-,
X est sélectionné parmi le groupe constitué d'une liaison directe ; -(OCH₂)q-(q représente un entier compris entre 0 et 10) ; d'alkylène substitué ou non substitué portant 1 à 20 atomes de carbone ; d'alkénylène substitué ou non substitué portant 2 à 20 atomes de carbone ; de cycloalkylène substitué ou non substitué portant 5 à 12 atomes de carbone ; d'arylène substitué ou non substitué portant 6 à 40 atomes de carbone ; d'aralkylène substitué ou non substitué portant 7 à 15 atomes de carbone ; et d'alkynylène substitué ou non substitué portant 2 à 20 atomes de carbone ;
R₃, R₄ et R₅ sont chacun indépendamment sélectionnés parmi le groupe constitué d'hydrogène ; d'halogène ; d'alkyle linéaire ou ramifié portant 1 à 20 atomes de carbone ; de vinyle ou d'alkényle linéaire ou ramifié portant 2 à 20 atomes de carbone ; de cycloalkyle substitué ou non substitué par des hydrocarbures et portant 5 à 12 atomes de carbone ; d'aryle substitué ou non substitué par des hydrocarbures et portant 6 à 40 atomes de carbone ; d'aralkyle substitué ou non substitué par des hydrocarbures et portant 7 à 15 atomes de carbone ; et d'alkynyle portant 2 à 20 atomes de carbone, 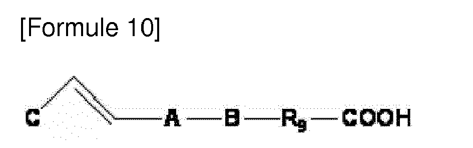 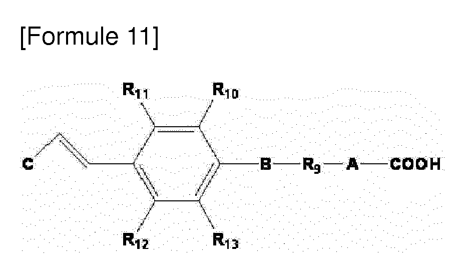 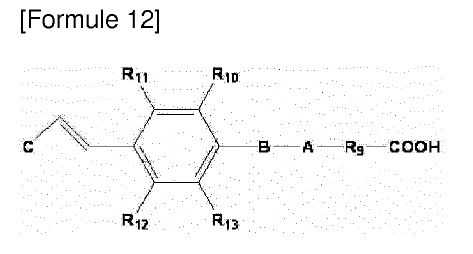 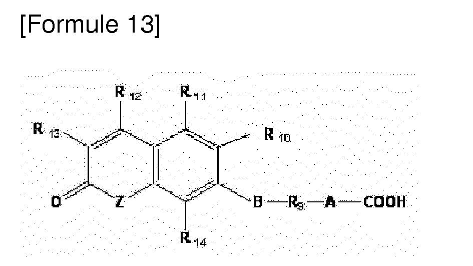
dans lesquelles A, B, C, Z, R₉, R₁₀, R₁₁, R₁₂, R₁₃ et R₁₄ sont tels que définis dans les Formules 4 à 7.

4. Procédé de production d'un composé d'ester selon le mode opératoire de la revendication 3,
dans lequel la composition catalysante d'estérification est utilisée selon le rapport de 0,01 à 20 sur la base d'un nombre de moles du composé qui est ajouté en un volume plus petit parmi les composés d'alcool et d'acide carboxylique.

5. Procédé de production d'un composé d'ester selon le mode opératoire de la revendication 3, dans lequel C des Formules 4 à 6 est sélectionné parmi le groupe constitué des Formules suivantes : 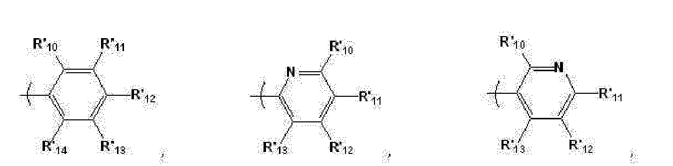 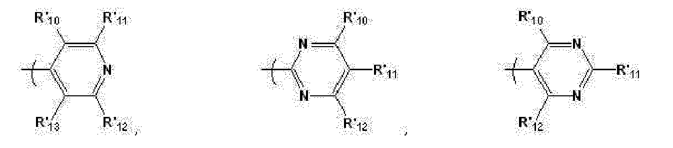 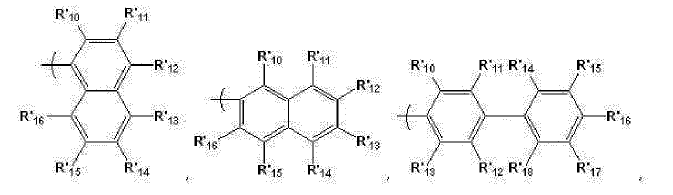 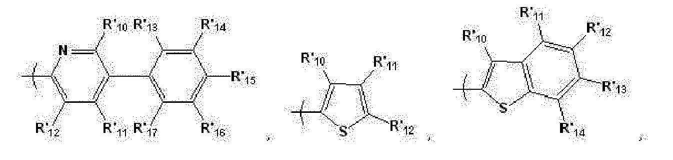 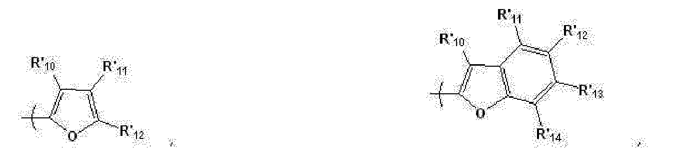 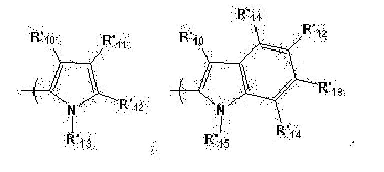 dans lesquelles R'₁₀, R'₁₁, R'₁₂, R'₁₃, R'₁₅, R'₁₆, R'₁₇ et R'₁₈ sont chacun indépendamment sélectionnés parmi le groupe constitué d'hydrogène ; d'halogène ; d'alkyle substitué ou non substitué portant 1 à 20 atomes de carbone ; d'alcoxy substitué ou non substitué portant 1 à 20 atomes de carbone ; d'aryloxy substitué ou non substitué portant 6 à 30 atomes de carbone ; d'aryle substitué ou non substitué portant 6 à 40 atomes de carbone.

6. Procédé de production d'un composé d'ester selon le mode opératoire de la revendication 3, dans lequel l'alcool comprend un ou plusieurs éléments sélectionnés parmi le groupe constitué d'alcools primaires aliphatiques dont méthanol, éthanol, n-propanol, n-butanol, n-hexanol, n-heptanol, n-octanol, n-décanol, n-dodécanol, alcool stéarylique, 2-éthylhexane-1-ol et alcool de néopentyle ; d'alcools primaires aromatiques de l'alcool benzylique ; d'alcools secondaires aliphatiques dont l'alcool isopropylique, l'alcool s-butyle et 1-méthylhexane-1-ol ; d'alcools secondaires alicycliques dont le cyclohexanol et le 2-adamanthirol ; d'alcools tertiaires dont l'alcool t-butyle, 1-adamanthirol, phénol, o-crésol, m-crésol, p-crésol, 3,5-diméthylphénol, α-naphtol et β-naphtol ; et d'alcools polyvalents dont éthylène glycol, propylène glycol, triméthylène glycol, 1,4-butanediol, 1,5-pentanediol, 1,6-hexanediol, 1,7-heptanediol, 1,8-octanediol, pinacol, néopentyle glycol, triméthylol propane, triméthylol éthane, pentaérythritol, dipentaérythritol, sorbitol et alcool polyvinylique.

7. Procédé de production d'un composé d'ester selon le mode opératoire de la revendication 3, dans lequel le composé d'acide carboxylique comprend un ou plusieurs éléments sélectionnés parmi le groupe constitué d'acides gras, dont un acide acétique, un acide propionique, un acide n-butyrique, un acide isobutyrique, un acide n-valérique, un acide isovalérique, un acide méthyléthyle acétique, un acide triméthyle acétique, un acide caproïque, un acide énantique, un acide caprylique, un acide pélargonique, un acide caprique, un acide undécylique, un acide laurique, un acide tridécylique, un acide myristique, un acide pentadécylique, un acide palmitique, un acide heptadécylique, un acide stéarique, un acide acrylique, un acide crotonique, un acide isocrotonique, un acide undécylénique, un acide oléique, un acide élaidique, un acide érucique, un acide brassidique, un acide sorbique, un acide linoléique et un acide linolénique ; des acides aromatiques tels que l'acide benzoïque ; des acides dicarboxyliques tels que l'acide malonique, un acide succinique, un acide glutarique, un acide adipique, un acide pimélique, un acide subérique, un acide azélaïque, un acide sébacique, un acide dodécanédioïque, un acide fumarique, un acide maléïque, un acide mésaconique, un acide citraconique, un acide phtalique, un acide téréphtalique, un acide isophtalique et un acide de diphényle éther-4,4'-di carboxylique ; des acides tricarboxyliques dont un acide butane-1,2,4-tricarboxylique, un acide cyclohexane-1,2,3-tricarboxylique, un acide benzène-1,2,4-tricarboxylique et un acide naphtalène-1,2,4-tricarboxylique ; et des acides tétracarboxyliques dont un acide butane-1,2,3,4-tétracarboxylique et un acide cyclobutane-1,2,3,4-tétracarboxylique, un acide benzène-1,2,4,5-tétracarboxylique, un acide 3,3',4,4'-benzophénone tétracarboxylique et un acide 3,3',4,4'-diphényléther tétracarboxylique.

8. Procédé de production d'un composé d'ester, le procédé comprenant les étapes consistant à :
estérifier un acide ω-hydroxycarboxylique qui possède chacun indépendamment un groupe hydroxyle et un groupe carboxy aux deux extrémités dans une molécule en utilisant la composition catalysante selon la revendication 1.

9. Procédé de production d'un composé d'ester selon le mode opératoire de la revendication 8, dans lequel l'acide ω-hydroxycarboxylique contient l'acide ω-hydroxy undécanoïque, l'acide hydroxy dodécanoïque, l'acide p-hydroxy benzoïque, l'acide m-hydroxy benzoïque, l'acide 6-hydroxynaphtalène-2-carboxylique, l'acide 4-(p-hydroxyphénoxy)benzoïque, l'acide 3-(p-hydroxyphénoxy)benzoïque, l'acide 4-(m-hydroxyphénoxy)benzoïque et l'acide 3-(m-hydroxyphénoxy)benzoïque.
